# EUROPEAN PATENT SPECIFICATION

(11) **EP 2 244 745 B1**
(45) Date of publication and mention of the grant of the patent: **25.03.2015**
(21) Application number: 09713457.1
(22) Date of filing: 23.02.2009
(51) Int. Cl.: A61L 15/34, A61L 15/42

(54) **ABSORBENT ARTICLE COMPRISING LOTION COMPOSITION COMPRISING A COOLING AGENT**
SAUGFÄHIGER ARTIKEL MIT EINER KÜHLMITTELHALTIGEN LOTION
ARTICLE ABSORBANT COMPRENANT UNE COMPOSITION DE LOTION COMPRENANT UN AGENT DE REFROIDISSEMENT

(30) Priority: 22.02.2008 US 30644
(43) Date of publication of application: 03.11.2010
(73) Proprietor: The Procter & Gamble Company, Cincinnati, OH 45202 (US)
(72) Inventor: WARREN, Raphael, Cincinnati, Ohio 45237 (US); DECKNER, George, Endel, Cincinnati, Ohio 45249 (US); HAUGHT, John, Christian, West Chester, Ohio 45069 (US)
(74) Representative: Briatore, Andrea
(86) International application number: PCT/US2009/034810
(87) International publication number: WO 2009/105740

(56) References cited:
- WO-A1-2005/049553
- US-A1- 2004 081 680
- US-A1- 2007 233 026
- US-B2- 6 749 860

## Description

### FIELD OF THE INVENTION

The present invention relates to a feminine care absorbent article comprising a lotion composition comprising a cooling agent.

### BACKGROUND OF THE INVENTION

Disposable absorbent articles, such as diapers, training pants, and catamenial devices having lotioned topsheets are known. Lotions of various types are known to provide various skin benefits, such as prevention or treatment of diaper rash. These lotions can be applied to the topsheet of absorbent articles, for example, and can be transferred to the skin of the wearer during use.

Certain wearers of disposable absorbent articles, such as catamenial devices, prefer products that can provide sensorial benefits, in addition to fluid absorbency benefits. A sensorial benefit desired by some wearers is a cooling sensation that can convey a feeling of freshness to the weaver. There has thus been a desire to create an absorbent article, such as a catamenial

Previous attempts have been made to incorporate conventional cooling agents, such as menthyl lactate or N-ethyl p-menthanecarboxamide (also known as "WS-3"), in absorbent articles, such as catamenial devices. See, e.g., US 2004/00826654 A1 and US 2004/0081680 A1. Due to their volatile property, these materials are generally placed in products that can directly reach the sensory receptors in the skin or mucosal tissue. This is achieved either through direct contact with the target tissue (i.e., oral rinse, nasal spray, massage oil). In the context of an absorbent article these materials are generally loaded into the core of the product. Nonetheless, these conventional cooling agents remain highly volatile such that it can be difficult for such materials in an absorbent article to remain stable upon shipment and storage, especially when exposed to high temperatures during shipment and storage. Other cooling agents have recently been identified that have lower volatility. However, this presents a problem of delivery to the epidermal cooling receptors. Furthermore, some of these cooling agents can be unacceptable to the wearer due to the psychological profile associated with the odor of the cooling agent.

An absorbent article comprising a sensation member that includes a sensate to provide a sensation of temperature change greater than an actual temperature change to the wearer's skin in response to a urination event is described in US 2007/0233026 A1.

N-substituted p-menthane carboxamides that give a cooling sensation to the mouth and skin are described in WO 2005/049553 A1.

A desire thus remains to develop an absorbent article product that can provide a sensorial benefit to the wearer, such as a cooling sensation, that does not suffer from stability problems upon shipment and storage of the absorbent article product and can be delivered to the skin of the wearer.

### SUMMARY OF THE INVENTION

The present invention relates to an absorbent article comprising: a topsheet; a backsheet; an absorbent core disposed between said topsheet and said backsheet; and a lotion composition disposed on one or more layers of said absorbent article, said lotion composition comprising: a carrier; and a cooling agent comprising a N-substituted p-menthane carboxamide material selected from the group consisting of N-(4-cyanomethylphenyl) p-enthanecarboxamide, N-(4-sulfamoylphenyl) p-menthanecarboxamide, N-(4-cyanophenyl) p-enthanecarboxamide, N-(4-acetylphenyl) p-menthanecarboxamide, N-(4-hydroxymethylphenyl) p-menthanecarboxamide, N-(3-hydroxy-4-methoxyphenyl) p- menthanecarboxamide, and mixtures thereof.

In an embodiment, the absorbent article comprises a topsheet, a backsheet, an absorbent core disposed between the topsheet and the backsheet, and a lotion composition disposed on one or more layers of the absorbent article, wherein the lotion composition comprises a cooling agent having an enthalpy of vaporization of at least 71 kJ/mol at 101.325 kPa (760 Torr).

The cooling agent is preferably substantially odorless (e.g. having no perceptible odor or only a light or faint perceptible odor).

The absorbent articles of the present invention comprising such cooling agents provide a beneficial cooling sensation to the skin of the wearer of the absorbent article.

### BRIEF DESCRIPTION OF THE DRAWING

The Figure is a top view of an absorbent article comprising a topsheet, backsheet, and an absorbent core, with a lotion composition applied thereto.

### DETAILED DESCRIPTION OF THE INVENTION

As used herein, the term "absorbent article" refers to devices that absorb and contain body exudates, primarily menses. The term "disposable" is used herein to describe absorbent articles which are not intended to be laundered or otherwise restored or reused as an absorbent article after a single use. Examples of absorbent articles include feminine hygiene garments such as sanitary napkins, pantiliners, interlabial devices, hemorrhoid pads, and the like.

The absorbent articles herein are utilized by consumers who can detect and react to sensate materials, such as the cooling agents described herein, and are typically adults.

Disposable absorbent articles and components thereof, including the topsheet, backsheet, absorbent core, and any individual layers of these components, have a body surface and a garment surface. As used herein, "body surface" means that surface of the article or component which is intended to be worn toward or adjacent to the body of the wearer, while the "garment surface" is on the opposite side and is intended to be worn toward or placed adjacent to the wearer's undergarments when the disposable absorbent article is worn.

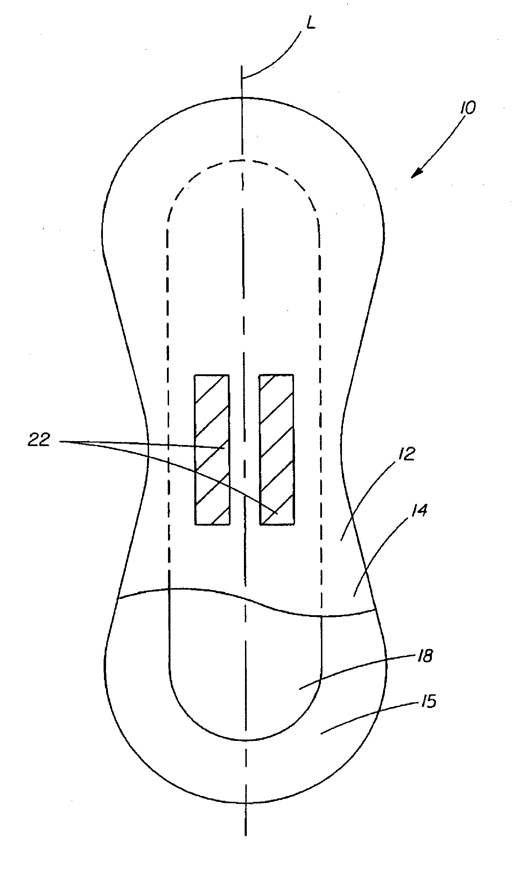

FIG. 1 shows a catamenial device 10, that can be a sanitary napkin or pantyliner, having a body-contacting surface 12 comprising a topsheet 14, a liquid impervious backsheet 16 joined to the topsheet 14, an absorbent core 18. The sanitary napkin 10 has a longitudinal axis L and may also be provided with additional features commonly found in napkins, including "wings" or "flaps" (not shown) as is known in the art, and , and/or a fluid acquisition layer to promote fluid transport to the absorbent core 18. Likewise, the topsheet of the sanitary napkin can have various optional characteristics, as is known in the art. For example, the topsheet 14 can have channels embossed therein to direct fluid flow, and can have apertures therethrough to aid in fluid acquisition. The topsheet 14 of the catamenial device 10 of the present invention has a lotion composition 22 disposed onto the topsheet.

The topsheet is preferably compliant, soft feeling, and non-irritating to the wearers skin and hair. Further, the topsheet is liquid pervious, permitting liquids (e.g., menses and/or urine) to readily penetrate through its thickness. A suitable topsheet may be manufactured from a wide range of materials such as woven and nonwoven materials (e.g., a nonwoven web of fibers); polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers) or from a combination of natural and synthetic fibers. When the topsheet comprises a nonwoven web, the web may be manufactured by a wide number of known techniques. For example, the web may be spunbonded, carded, wet-laid, melt-blown, hydroentangled, combinations of the above, or the like.

The backsheet is impervious to liquids (e.g., menses and/or urine) and is preferably manufactured from a thin plastic film, although other flexible liquid impervious materials may also be used. As used herein, the term "flexible" refers to materials which are compliant and will readily conform to the general shape and contours of the human body. The backsheet prevents the exudates absorbed and contained in the absorbent core from wetting articles which contact the absorbent article such as bedsheets, pants, pajamas and undergarments. The backsheet may thus comprise a woven or nonwoven material, polymeric films such as thermoplastic films of polyethylene or polypropylene, or composite materials such as a film-coated nonwoven material.

The backsheet and the topsheet are positioned adjacent the garment surface and the body surface, respectively, of the absorbent core. The absorbent core can be joined with the topsheet, the backsheet, or both in any manner as is known by attachment means (not shown in FIG. 1) such as those well known in the art. However, embodiments of the present invention are envisioned wherein portions of the entire absorbent core are unattached to either the topsheet, the backsheet, or both.

In one embodiment, the topsheet of catamenial device 10 is made of a hydrophobic material. Therefore, if the topsheet is a nonwoven, the constituent fibers are preferably hydrophobic. Fibers are considered to be hydrophobic if film sheets formed from the polymers of the fibers would exhibit contact angles with water greater than 60 degrees, more preferably 75 degrees, and even more preferably greater than about 90 degrees. Contact angles as a measure of hydrophobicity are well known in the art, and methods for measuring contact angles are equally well known. As is well known, contact angles greater than about 90 degrees are considered hydrophobic, and contact angles less than 90 degrees are often considered hydrophilic. As used herein, however, contact angles of 60 degrees or greater are considered hydrophobic.

In another embodiment, the topsheet of catamenial device 10 is made of a hydrophilic material.

The cooling compounds may be prepared by reacting an arylalkylamine derivative with an appropriate acid chloride or carbonyl chloride. The carbonyl chloride can be prepared from 1-menthol ((1R,2S,5R)-2-isopropyl-5-methylcyclohexanol).

The particular N-substituted p-menthane carboxamide cooling agents according to the present invention tend to have lower volatility than other more conventional cooling agents, such as those recited in the previous paragraph. Lower volatility cooling agents are especially useful for incorporation in the absorbent articles of the present invention. It is believed that such lower volatility cooling agents are more stable in the finished product upon shipment and storage of the product, wherein the absorbent articles are potentially exposed to higher temperatures. The more conventional and more highly volatile cooling agents tend to be unstable in such shipping and storage environments unless steps are taken to increase the stability such as hermetically sealing the absorbent articles to avoid exposure to the surrounding environment. Such packaging, however, significantly increases costs and results in a product that is less consumer-friendly, e.g. it is more difficult to open and difficult to dispose. Alternatively, the more conventional cooling agents would have to be used at much higher levels in the absorbent article to offset the amount of cooling agent lost during shipping and storage due to volatilization of the cooling agent, thereby resulting in higher costs.

In one embodiment, the particular cooling agent utilized herein will have an enthalpy of vaporization of at least 71 kJ/mol at 760 Torr, or at least 73 kJ/mol at 760 Torr, or at least 75 kJ/mol at 760 Torr. The enthalpy of vaporization is a calculated value determined by using, for example, Advanced Chemistry Development (ACD/Labs) Software V9.04 for Solaris (© 1994-2008 ACD/Labs). For example, N-(4-cyanomethylphenyl)-p-menthanecarboxamide has an enthalpy of vaporization of approximately 75.8 kJ/mol at 101.325 kPa (760 Torr); 3-(1-menthoxy)-2-methylpropane-1,2-diol (TPG-1) has an enthalpy of vaporization of approximately 71.6 kJ/mol at 101.325 kPa (760 Torr); and 1-(2-hydroxyphenyl)-4-(3-nitrophenyl)-3,6-dihydropyrimidin-2-one (ICILIN or AG-3-5) has an enthalpy of vaporization of approximately 91.9 kJ/mol at 101.325 kPa (760 Torr).

The particular cooling materials, such as the particular N-substituted p-menthane carboxamides, recited above can be utilized alone, in combination with each other, and/or in combination with more traditional cooling materials known in the art, for example, menthol, menthone, isopulegol, N-ethyl p-menthanecarboxamide (WS-3), N,2,3-trimethyl-2-isopropylbutanamide (WS-23), menthyl lactate (Frescolat^{®} ML), menthone glycerine acetal (Frescolat^{®} MGA), mono-menthyl succinate (Physcool^{®}), mono-menthyl glutarate, O-menthyl glycerine (CoolAct^{©} 10), menthyl-N,N-dimethylsuccinamate or 2-sec-butylcyclohexanone (Freskomenthe^{®}). Such conventional cooling materials tend to have an enthalpy of vaporization of less than 71 kJ/mol at 101.325 kPa (760 Torr), and preferably less than 65 kJ/mol at 101.325 kPa (760 Torr). For example, N-ethyl p-menthanecarboxanude (WS-3), N,2,3-trimethyl-2-isopropylbutanamide (WS-23), menthol, menthyl lactate, ethyl 3-(p-menthane-3-carboxamide) acetate (WS-5), menthone glycerine acetal, menthoxypropane-1,2-diol, isopulegol, p-menthane-3,8-diols, N,N-dimethyl menthyl succinamide, (3S,3aR,3bR,4S,7R,7aR)-Octahydro-3,7-dimethyl-4-(1-methylethyl)-1H-cyclopenta[1,3] cyclopropa[1,2]benzen-3-ol (Cubebol^{®}), and menthyl PCA, have enthalpy of vaporization values of approximately 58.4 kJ/mol at 101.325 kPa (760 Torr), approximately 47 kJ/mol at 101.325 kPa (760 Torr), approximately 52.5 kJ/mol at 101.325 kPa (760 Torr), approximately 63.1 kJ/mol at 101.325 kPa (760 Torr), approximately 64 kJ/mol at 101.325 kPa (760 Torr), approximately 65.5 kJ/mol at 101.325 kPa (760 Torr), approximately 70.5 kJ/mol at 101.325 kPa (60 Torr), approximately 50.4 kJ/mol at 101.325 kPa (760 Torr), approximately 58.7 kJ/mol at 101.325 kPa (760 Torr), approximately 63.4 kJ/mol at 101.325 kPa (760 Torr), approximately 60.2 kJ/mol at 101.325 kPa (760 Torr), and approximately 66.1 kJ/mol at 101.325 kPa (760 Torr), respectively.

The particular cooling agents herein, such as the N-substituted p-menthane carboxamide cooling agent, can be blended in a carrier to be described below at a concentration ranging from about 0.0001% to about 10.0%, from about 0.0001% to about 3.0%, from about 0.001% to about 1.0%, or from about 0.01% to about 1.0%, by weight of the lotion composition. The cooling agent can also be prepared in a premix in an oil diluent. Nonetheless, the final concentration of the active principal will fall in the range described above.

The lotion composition of the present invention further comprises a carrier. The carrier helps to deliver the cooling agents of the present invention to the skin of the wearer of the absorbent article. The carrier can be included in the compositions as an individual carrier or a combination of carrier ingredients. The carrier can be a liquid, solid, or semisolid carrier material, or a combination of these materials, and preferably forms a homogenous mixture or solution at selected processing temperatures for the resultant carrier system and at processing temperatures for combining the carrier with the cooling agents in formulating the lotion compositions herein. Processing temperatures for the carrier system typically range from about 60°C to about 90°C, more typically from about 70°C to about 85°C, even more typically from about 65°C to about 80°C.

The lotion compositions of the present invention can comprise the carrier at a total carrier concentration ranging from about 60% to about 99.9%, preferably from about 70% to about 98%, more preferably from about 80% to about 97%, by weight of the lotion composition. Suitable carrier compounds include petroleum-based hydrocarbons having from about 4 to about 32 carbon atoms, fatty alcohols having from about 12 to about 24 carbon atoms, polysiloxane compounds, fatty acid esters, alkyl ethoxylates, lower alcohols having from about 1 to about 6 carbon atoms, low molecular weight glycols and polyols, fatty alcohol ethers having from about 12 to about 28 carbon atoms in their fatty chain, lanolin and its derivatives, glyceride and its derivatives including acetoglycerides and ethoxylated glycerides of C12-C28 fatty acids, and mixtures thereof. Alternatively or in combination with, the carrier may also be composed of polysiloxane compounds non-limiting examples include dimethicones (1-100,000,000 centistoke), cyclomethicones, alkylated silicones (hair conditioning agents), silicone gums, silicone gels, silicone waxes, copolymers of silicone (vinyl dimethicone polymers, phenyl vinyl dimethicone polymers, alkylated silicone polymers, polyethylene oxide / silicone copolymers, polyethylene oxide / alkyl silicone copolymers), and mixtures thereof.

Nonlimiting examples of suitable petroleum-based hydrocarbons having from about 4 to about 32 carbon atoms include mineral oil, petrolatum, isoparaffins, various other branched chained hydrocarbons, and combinations thereof. Mineral oil is also known as "liquid petrolatum", and usually refers to less viscous mixtures of hydrocarbons having from about 16 to about 20 carbon atoms. Petrolatum is also known as "mineral wax", "petroleum jelly", and "mineral jelly", and usually refers to more viscous mixtures of hydrocarbons having from about 16 to about 32 carbon atoms. An example of commercially available petrolatum include petrolatum sold as Protopet® 1S which is available from the Witco Corporation located in Greenwich, Connecticut.

Other carriers suitable herein can include oils or fats such as natural oils or fats, or natural oil or fat derivatives, in particular of plant or animal origin. Non-limiting examples include avocado oil, apricot oil, apricot kernel oil, babassu oil, borage oil, borage seed oil, calendula oil, camellia oil, canola oil, carrot oil, cashew nut oil, castor oil, chamomile oil, cherry pit oil, chia oil, coconut oil, cod liver oil, corn oil, corn germ oil, cottonseed oil, eucalyptus oil, evening primrose oil, grape seed oil, hazelnut oil, jojoba oil, juniper oil, kernel oil, linseed oil, macadamia oil, meadowfoam seed oil, menhaden oil, mink oil, moringa oil, mortierella oil, olive oil, palm oil, palm kernel oil, peanut oil, peach kernel oil, rapeseed oil, rose hip oil, safflower oil, sandlewood oil, sesame oil, soybean oil, sunflower oil, sunflower seed oil, sweet almond oil, tall oil, tea tree oil, turnip seed oil, walnut oil, wheat germ oil, zadoary oil, or the hardened derivatives thereof. Hardened oils or fats from vegetal origin can include, e.g. hardened castor oil, peanut oil, soya oil, turnip seed oil, cottonseed oil, sunflower oil, palm oil, kernel oil, linseed oil, corn oil, olive oil, sesame oil, cocoa butter, shea butter and coconut oil.

Other non-limiting examples of fats and oils include: butter, C12-C18 acid triglyceride, camellia oil, caprylic/capric/lauric triglyceride, caprylic/capric/linoleic triglyceride, caprylic/capric/stearic triglyceride, caprylic/capric triglyceride, cocoa butter, C10-C18 triglycerides, egg oil, epoxidized soybean oil, glyceryl triacetyl hydroxystearate, glyceryl triacetyl ricinoleate, glycosphingolipids, human placental lipids, hybrid safflower oil, hybrid sunflower seed oil, hydrogenated castor oil, hydrogenated castor oil laurate, hydrogenated coconut oil, hydrogenated cottonseed oil, hydrogenated C12-C18 triglycerides, hydrogenated fish oil, hydrogenated lard, hydrogenated menhaden oil, hydrogenated mink oil, hydrogenated orange roughy oil, hydrogenated palm kernel oil, hydrogenated palm oil, hydrogenated peanut oil, hydrogenated shark liver oil, hydrogenated soybean oil, hydrogenated tallow, hydrogenated vegetable oil, lanolin and lanolin derivatives, lanolin alcohol, lard, lauric/palmitic/oleic triglyceride, lesquerella oil, maleated soybean oil, meadowfoam oil, neatsfoot oil, oleic/linoleic triglyceride, oleic/palmitic/lauric/myristic/linoleic triglyceride, oleostearine, olive husk oil, omental lipids, orange roughy oil, pengawar djambi oil, pentadesma butter, phospholipids, pistachio nut oil, placental lipids, rapeseed oil, rice bran oil, shark liver oil, shea butter, sphingolipids, tallow, tribehenin, tricaprin, tricaprylin, triheptanoin, trihydroxymethoxystearin, trihydroxystearin, triisononanoin, triisostearin, trilaurin, trilinolein, trilinolenin, trimyristin, trioctanoin, triolein, tripalmitin, trisebacin, tristearin, triundecanoin, vegetable oil, wheat bran lipids, and the like, as well as mixtures thereof.

Other suitable carriers include mono- or di-glycerides, such as those derived from saturated or unsaturated, linear or branch chained, substituted or unsubstituted fatty acids or fatty acid mixtures. Examples of mono- or diglycerides include mono- or di-C₁₂₋₂₄fatty acid glycerides, specifically mono- or di-C₁₆₋₂₀fatty acid glycerides, for example glyceryl monostearate, glyceryl distearate.

Carriers can also include esters of linear C₆-C₂₂-fatty acids with branched alcohols.

The carrier of the present invention can also include sterols, phytosterols, and sterol derivatives. Sterols and sterol derivatives that can be used in the lotion compositions of the invention include, but are not limited to: β-sterols having a tail on the 17 position and having no polar groups for example, cholesterol, sitosterol, stigmasterol, and ergosterol, as well as, C10-C30 cholesterol/lanosterol esters, cholecalciferol, cholesteryl hydroxystearate, cholesteryl isostearate, cholesteryl stearate, 7-dehydrocholesterol, dihydrocholesterol, dihydrocholesteryl octyldecanoate, dihydrolanosterol, dihydrolanosteryl octyldecanoate, ergocalciferol, tall oil sterol, soy sterol acetate, lanasterol, soy sterol, avocado sterols, "AVOCADIN" (trade name of Croda Ltd of Parsippany, N.J.), sterol esters and similar compounds, as well as mixtures thereof. A commercially available example of phytosterol is GENEROL 122 N PRL refined soy sterol from Cognis Corporation of Cincinnati, Ohio.

Nonlimiting examples of suitable fatty alcohols having from about 12 to about 24 carbon atoms include saturated, unsubstituted, monohydric alcohols or combinations thereof, which have a melting point less than about 110°C, preferably from about 45°C to about 110°C. Specific examples of fatty alcohol carriers for use in the lotion compositions of the present invention include, but are not limited to, cetyl alcohol, stearyl alcohol, cetearyl alcohol, behenyl alcohol, arachidyl alcohol, lignocaryl alcohol, and combinations thereof. Examples of commercially available cetearyl alcohol is Stenol 1822 and behenyl alcohol is Lanette 22, both of which are available from the Cognis Corporation located in Cincinnati, Ohio.

Nonlimiting examples of suitable fatty acid esters include those fatty acid esters derived from a mixture of C₁₂-C₂₈ fatty acids and short chain (C₁-C₈, preferably C₁-C₃) monohydric alcohols preferably from a mixture of C₁₆-C₂₄ saturated fatty acids and short chain (C₁-C₈, preferably C₁-C₃) monohydric alcohols. Suitable fatty acid esters can also be derived from esters of longer chain fatty alcohols (C₁₂-C₂₈, preferably C₁₂-C₁₆) and shorter chain fatty acids such as lactic acid, specific examples of which include lauryl lactate and cetyl lactate. Representative examples of suitable fatty acid esters include methyl palpitate, methyl stearate, isopropyl laurate, isopropyl myristate, isopropyl palmitate, ethylhexyl palpitate, stearyl stearate, palmityl stearate, stearyl behenate, cetyl stearate, cetyl behenate, cetyl palpitate, cetearyl behenate, behenyl behenate, stearyl heptanoate, stearyl octanoate, myristyl myristate, myristyl isostearate, myristyl oleate, cetyl isostearate, cetyl oleate, stearyl isostearate, stearyl oleate, isostearyl myristat, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl isostearate, behenyl oleate, erucyl isostearate, and mixtures thereof.

Nonlimiting examples of suitable alkyl ethoxylates include C₁₂-C₂₂ fatty alcohol ethoxylates having an average degree of ethoxylation of from about 2 to about 30. Nonlimiting examples of suitable lower alcohols having from about 1 to about 6 carbon atoms include ethanol, isopropanol, butanediol, 1,2,4-butanetriol, 1,2 hexanediol, ether propanol, and mixtures thereof. Nonlimiting examples of suitable low molecular weight glycols and polyols include ethylene glycol, polyethylene glycol (e.g., Molecular Weight 200-600 g/mole), butylene glycol, propylene glycol, polypropylene glycol and mixtures thereof. A more detailed description of carrier ingredients including suitable hydrocarbons, polysiloxane compounds, and fatty alcohol ethoxylates can be found in U.S. Patent No. 5,643,588, issued July 1, 1997 to Roe et al. entitled "Diaper Having A Lotioned Topsheet".

Suitable carriers further encompass waxes. As used herein, the term 'wax' refers to oil soluble materials that have a waxy constituency and have a melting point or range of above ambient temperature, in particular above 25°C. Waxes are materials that have a solid to semisolid (creamy) consistency, crystalline or not, being of relative low viscosity a little above their liquefying point. Suitable waxes which can be incorporated into the lotion composition include animal, vegetable, mineral or silicone based waxes which may be natural or synthetic, and including mixtures thereof. Waxes can include but are not limited to: natural waxes from vegetal origin, such as bayberry wax, beeswax, candelilla wax, carnauba, ceresin, purcelline, shea butter, cocoa butter, Japan wax, jojoba wax, lanolin wax, esparto gras wax, cork wax, guaruma wax, rice shoot wax, ouricury wax, mink wax, montan wax, rice bran wax, spent grain wax, spermaceti wax, steryl dimethicone, sunflower wax, ceresine wax, sugar cane wax, carnauba wax, candelilla wax, fruit-derived waxes, such as orange wax, lemon wax, grapefruit wax and bayberry wax, and the like; and waxes from animal origin such as beeswax, woolwax, bear fat, shellac wax, and the like. Natural waxes further comprise mineral waxes such as ceresin and ozokerite waxes. Synthetic waxes comprise petroleum-based waxes, such as certain carrier materials described hereinbefore, such as paraffin, vaseline, petrolatum, micro wax, and microcrystalline wax. Further suitable synthetic waxes are polyalkylene and polyethyleneglycol waxes, e.g. polyethylene wax; waxes based on chlorinated naphtalenes such as 'Halowax', synthetic hydrocarbon waxes, and the like, PEG-6 beeswax, PEG-8 beeswax, C30 alkyl dimethicone, synthetic beeswax, synthetic candelilla wax, synthetic carnuba wax, synthetic japan wax, synthetic jojoba wax, motan acid wax, motan wax, ouricury wax, rezowax, including mixtures thereof. Further suitable waxes are chemically modified waxes, in particular hardened or hydrogenated waxes such as, for example, hydrogenated cottonseed oil, hydrogenated jojoba oil, hydrogenated jojoba wax, hydrogenated microcrystalline wax, hydrogenated rice bran wax, Montan-ester waxes, Sasol waxes, jojoba esters, and the like.

Other wax components can be certain fats (including mono-, di- and triglycerides and fatty acid alkylesters), fatty alcohols, fatty acids, including substituted fatty acids (in particular hydroxy substituted fatty acids, for example, 12- hydroxystearic acid), dialkyl(ene)ethers, dialkyl(ene) carbonates, dicarboxylic acids (in particular the C₁₆-C₄₀- dialkylesters of dicarboxylic acids, e.g. the C₁₆-C₄₀- alkyl stearates, C₁₈-C₃₈-alkylhydroxystearyl stearates or C₂₀-C₄₀- alkyl erucates) and hydroxy fatty alcohols. Still further wax components are selected from the group of aromatic carbonic acids, tricarboxylic acids, or from the group of lactides of long-chained hydroxycarbonic acids. Myristyl lactate is a suitable carrier. Further wax components that can be used are C₃₀-C₅₀ alkyl bees wax; tri-C₁₆-C₄₀-alkyl citrates, e.g. tristearyl citrate, triisostearyl citrate, trilauryl citrate; ethyleneglycol di fatty acid esters, in particular the ethylene glycol di-C₁₂-C₃₀- fatty acid esters, e.g. ethylene glycol dipalmitate, ethyleneglycol distearate, and ethyleneglycol di(12- hydroxystearate).

Other suitable carriers include materials that act as solidifying agents, including some of the materials described hereinbefore. Suitable solidifying agent(s) in the lotion compositions of the present invention can function to help solidify the composition so that the composition is a solid at room temperature and has a melting point of at least 32°C. The solidifying agent may also provide a tackiness to the composition that improves the transfer by adhesion to the skin of the wearer. Depending on the solidifying agent selected, the solidifying agent can also modify the mode of transfer so that the composition tends to fracture or flake off instead of actually rubbing off onto the skin of the wearer which can lead to improved transfer to the skin. The solidifying agent may further function as an emollient, occlusive agent, moisturizer, barrier enhancer, viscosity enhancer and combinations thereof. The solidifying agents can be selected from alkyl siloxanes, polymers, hydrogenated vegetable oils having a melting point of 35°C or greater, fatty acid esters with a melting point of 35°C or greater, alkyl hydroxystearates, branched esters, alkoxylated alcohols and alkoxylated carboxylic acid. Additionally, the solidifying agents can be selected from animal, vegetable and mineral waxes and alkyl silicones. Examples of suitable solidifying agents include, but are not limited to, the following: alkyl silicones, alkyl trimethylsilanes, beeswax, behenyl behenate, behenyl benzoate, C24-C28 alkyl dimethicone, C30 alkyl dimethicone, cetyl methicone, stearyl methicone, cetyl dimethicone, stearyl dimethicone, cerotyl dimethicone, candelilla wax, carnuba, synthetic carnuba, PEG-12 carnauba, cerasin, hydrogenated microcrystalline wax, jojoba wax, microcrystalline wax, lanolin wax, ozokerite, paraffin, synthetic paraffin, cetyl esters, behenyl behenate, C20-C40 alkyl behenate, C2-C5 lactate, cetyl palmitate, stearyl palmitate, isosteryl behenate, lauryl behenate, stearyl benzoate, behenyl isostearate, cetyl myristate, cetyl octanoate, cetyl oleate, cetyl ricinoleate, cetyl stearate, decyl oleate, di C2-C5 alkyl fumerate, dibehenyl fumerate, myristyl lactate, myristyl lignocerate, myristyl myristate, myristyl stearate, lauryl stearate, octyidodecyl stearate; octyidodecyl stearoyl stearate, oleyl arachidate, oleyl stearate, tridecyl behenate, tridecyl stearate, tridecyl stearoyl stearate, pentaerythrityl tetrabehenate, penteerythritylhydrogenated rosinate, pentaerythrityl distearate, pentaerythrityltetraabeite, penteerythrityl tetracocoate, penteerythrityl tetraperlargonate, pentserythrityl tetrastearate, ethylene vinyl acetate, polyethylene, hydrogenated cottonseed oil, hydrogenated vegetable oil, hydrogenated squalene, hydrogenated coconut oil, hydrogenated jojoba oil, hydrogenated palm oil, hydrogenated palm kernel oil, hydrogenated olive oil, polyamides, metal stearates and other metal soaps, C30-C60 fatty alcohols, C20+ fatty amides, polypropylene, polystyrene, polybutane, polybutylene terephthalate, polydipentane, polypropylene, zinc stearate, dodecyl laurate, stearyl palmitate, octadecyl hexedecanoate, octadecyl palmitate, stearyl behenate, docosyl octanoate, tetradecyl-octadecanyl behenate, hexadecyl-cosanyl hexacosanate, shellac wax, glycol montanate, fluoranated waxes, C20-C40 alkyl hydroxystearyl stearate, and mixtures of such compounds.

The absorbent article of the present invention can optionally further comprise indicia to emphasize the cooling benefits or cooling sensation provided by the absorbent article. Such indicia can include graphics, patterns, and the like that comprise colors such as green, blue, or combinations thereof. The colors green, blue, or combinations thereof tend to visually communicate the cooling benefits or cooling sensation provided by the absorbent article to the consumer. Such indicia can be incorporated into the packaging for the absorbent articles or can be incorporated into the absorbent articles themselves, such as inidicia that is printed or impregnated into the components of the absorbent article (e.g. the topsheet or backsheet of the absorbent article).

The absorbent article of the present invention can optionally further comprise essential oil materials that help to connote the cooling benefits or cooling sensation provided by the absorbent article. Such essential oil materials can be incorporated into the absorbent article separate from the lotion composition or can be made part of the lotion composition. Non-limiting examples of suitable essential oil materials include Acorus gramineus, Anthemis nobilis, Artemisia dracunculus, Basil, Bergamot, Calamintha sylvatica, Caraway, Cedarwood, Chamomile, Cineol, Cinnamon, Cinnamon bark, Citrus aurantium, Clove, Cypress, Dill, Eucalyptus, Eugenol, Frankincense, Galangol, Geranium, Ginger, Hibiscus, Hop, Jasmine, Juniper, Laurus nobilis, Lavender, Lemon balm, Lemongrass, Lemon, Limonene, Linalool, Linalyl acetate, Lippia alba, Marjoram, Melissa, Myrrh, Neroli, Nutmeg, Passiflora, Patchouli, Peppermint, Pinene, Rose, Rosewood, Rosemary, Sage, Sandalwood, Spearmint, Sweet Fennel, Sweet Orange, Thyme, Valerian, Ylang ylang, Hibiscus, or mixtures thereof. Preferred essential oils associated with arousal include Cypress, Hibiscus, Juniper, Cineol, Citrus, Sweet Orange, and Rosemary. Preferred oils associated with a harmonizing effect include Lavender, Neroli, and Ylang ylang.

The particular essential oils herein, such as described above, can be blended in a carrier at a concentration ranging from about 0.0001% to about 10.0%, from about 0.0001% to about 3.0%, from about 0.0001% to about 0.1%, from about 0.001% to about 1%, or from about 0.01% to about 1.0%, by weight of the lotion composition. The essential oil can also be prepared in a premix in an oil diluent. Nonetheless, the final concentration of the essential oil will typically fall in the ranges described above.

The cooling agents of the present invention, other ingredients of the lotion composition, or the lotion composition itself can be encapsulated in a variety of suitable encapsulation materials. Non-limiting examples of suitable encapsulation materials are described in detail as "Perfume Carrier Materials" in US 7,186,680 at column 25, line 23 to column 28, line 52.

When applied to the outer surface of sanitary napkin topsheets, the lotion compositions of the present invention can be transferable to the wearer's skin by normal contact, wearer motion, and/or body heat, thereby providing a cooling sensation on the skin of the wearer.

The sanitary napkin topsheets of the present invention contain an effective amount of the lotion composition. As used herein, the term "effective amount of a lotion composition" refers to an amount of a particular lotion composition which, when applied to a sanitary napkin topsheet, will be effective in providing a cooling benefit or cooling sensation to the skin of the wearer. The effective amount of a lotion composition will depend, to a large extent, on the particular lotion composition used.

In preparing lotioned absorbent articles according to the present invention, the lotion composition can be applied to the outer surface (i.e., body facing surface) of the topsheet, but can also be applied to the inner surface of the topsheet or to any other component of the absorbent article. Any of a variety of application methods that evenly distribute the lotion composition can be used. Suitable methods include spraying, printing (e.g., flexographic printing), coating (e.g., gravure coating), extrusion, or combinations of these application techniques, e.g. spraying the lotion composition on a rotating surface, such as a calender roll, that then transfers the composition to the outer surface of the topsheet.

The manner of applying the lotion composition to the topsheet, or other component, can be such that the topsheet does not become saturated with the lotion composition. If the topsheet becomes saturated with the lotion composition, there is a greater potential for the lotion to block the topsheet openings, reducing the ability of the topsheet to transmit fluid to the underlying absorbent core. Also, saturation of the topsheet is not required to obtain the therapeutic and/or protective lotion benefits. Particularly suitable application methods will apply the lotion composition primarily to the outer surface of the topsheet.

The minimum level of lotion to be applied to the topsheet is an amount effective for providing a cooling benefit or cooling sensation to the skin of the wearer. The lotion composition is preferably applied to the topsheet of the present invention in an amount ranging from about 0.1mg/in² to about 75 mg/in² (mg of lotion per square inch of coated topsheet), preferably from about 0.5 mg/in² to about 60 mg/in², and more preferably from about 1 mg/in² to about 60 mg/in². Such relatively low levels of lotion composition are adequate to impart the desired cooling benefits, yet do not saturate the topsheet's absorbency and/or wettability properties.

The lotion composition may be applied to the entire surface of the topsheet or portions thereof. The lotion composition can be applied in a stripe aligned with and centered on the longitudinal centerline of the disposable absorbent article. The lotion composition can be applied in a plurality of stripes having uniform or non-uniform widths. Alternatively the lotion can be aligned with and centered in apposition to the longitudinal centerline.

The lotion composition can also be applied nonuniformly to the outer surface of the sanitary napkin topsheet. By "nonuniform" is meant that the amount, pattern of distribution, etc. of the lotion composition can vary over the topsheet surface. For example, some portions of the treated surface of the topsheet can have greater or lesser amounts of lotion composition, including portions of the surface that do not have any lotion composition on it. For example, the lotion composition can be applied on one region of the topsheet in the shape of a rectangle and/or a circle, and/or as mutliplicity of dots.

The lotion composition can be applied to the topsheet or other component at any point during assembly. For example, the lotion composition can be applied to the topsheet of the finished disposable absorbent product before it has been packaged. The lotion composition can also be applied to the topsheet before it is combined with the other raw materials to form a finished disposable absorbent product.

The lotion composition is typically applied from a melt thereof to the absorbent article. Since the lotion composition will typically melt at significantly above ambient temperatures, it is usually applied as a heated coating. Typically, the lotion composition is heated to a temperature in the range from about 35°C to about 100°C, preferably from 40°C to about 90°C, prior to being applied. Once the melted lotion composition has been applied, it is allowed to cool and solidify to form solidified coating or film on the surface of the topsheet or other component. Preferably, the application process is designed to aid in the cooling/set up of the lotion.

Lotion compositions of the present invention can be applied by printing methods, or continuous spray or extrusion as is known in the art, or as is described in US 5,968,025.

It can be preferred that the lotion be applied in a plurality of stripes parallel to the longitudinal axis of the absorbent article. This allows for both transfer of the lotion to a broader area of the vulva and improved fluid handling of the absorbent article.

In another embodiment, instead of (or in addition to) being applied to the topsheet of an absorbent article, the lotion composition can be applied to a wipe article that is supplied with the absorbent article (for example, as described in detail in US 5,569,230 or US 6,911,022). In another embodiment, the lotion composition can be provided as a stand-alone product in the form of a cream product that can be applied to the absorbent article or to the skin by hand. In another embodiment, the lotion composition can be provided as a stand-alone product in the form of a spray product that can be sprayed onto the absorbent article or the skin by the wearer of the absorbent article.

The following are non-limiting examples of the present invention.

Example I: The compositions exemplified hereinbelow in Table 1 are representative of carrier systems of the lotion compositions of the present invention. The carrier systems are generally prepared by combining, by weight, petrolatum and a fatty alcohol such as behenyl alcohol, and then heating the mixture while stirring to a temperature of about 80°C using a low speed propeller mixer. Next, viscosity or thickening agents, if present, are added to the mixture to shear mix the ingredients into a final carrier system. Suitable viscosity or thickening agents include beheneth-10, fumed silica, bentonite, and steareth-2, wherein the viscosity or thickening agents are used alone or in combination. The ingredients can be shear mixed at 11,000 revolutions per minute (rpm) using an IKA Ultra Turrax Shear Mixer.

Alternatively, when present, the petrolatum, fatty alcohol, and/or viscosity or thickening agent can be combined, heated with stirring at 80°C to melt the ingredients, and then mixed into a final carrier system using a high speed blade mixer such as the Tokusyu Kika TK Robo Mics which operates at 5,000 rpm.

**Table 1: Carrier Systems**

| Component | Carrier 1 | Carrier 2 | Carrier 3 | Carrier 4 | Carrier 5 |
|---|---|---|---|---|---|
| | (Wt. %) | (Wt. %) | (Wt. %) | (Wt. %) | (Wt. %) |
| Petrolatum¹ | 80.4 | 78.1 | 86.5 | 100 | 80 |
| Behenyl Alcohol² | 11 | 8.7 | 10 | | 20 |
| Cetearyl Alcohol³ | | | | | |
| Beheneth- 10⁴ | -- | 10 | | | |
| Fumed Silica⁵ | 3.6 | 3.2 | 3.5 | | |
| Polypropylene Glycol⁶ | 5 | | | | |

| | | | | | |
|---|---|---|---|---|---|
| Wt. % - weight percent 1 - Petrolatum available as Protopet® 1S from the Witco Corporation 2 - Behenyl alcohol available as Lanette 22 from the Cognis Corporation 3 - Cetearyl alcohol available as Stenol 1822 from the Cognis Corporation 4 - Beheneth-10 available as Mergital® B10 from the Cognis Corporation 5 - Fumed silica available as Cabosil® TS-720 from the Cabot Corporation 6 - Polypropylene glycol Mn 3,500 from Sigma Aldrich #202355 | | | | | |

Examples II-XV: The following Examples II-XV illustrated hereinbelow in Table 2 are representative of lotion compositions of the present invention that include the carriers identified in Table 1. The lotion compositions are prepared by formulating a premix solution of the zinc oxide skin treatment agent, if present, and adding the zinc oxide premix to the cooling agent and other skin treatment agents and any optional ingredients such as panthenol and glycerin, or by formulating a skin treatment solution of hexamidine and niacinamide skin treatment agents and any optional ingredients. The skin treatment solution is then added to a carrier such as those described in Table 1 to form the lotion composition, wherein the skin treatment solution and carrier are heated while stirring to a temperature of about 80°C. All ingredients are included by weight of the lotion composition. These lotion compositions provide a cooling sensation on the wearer's skin.

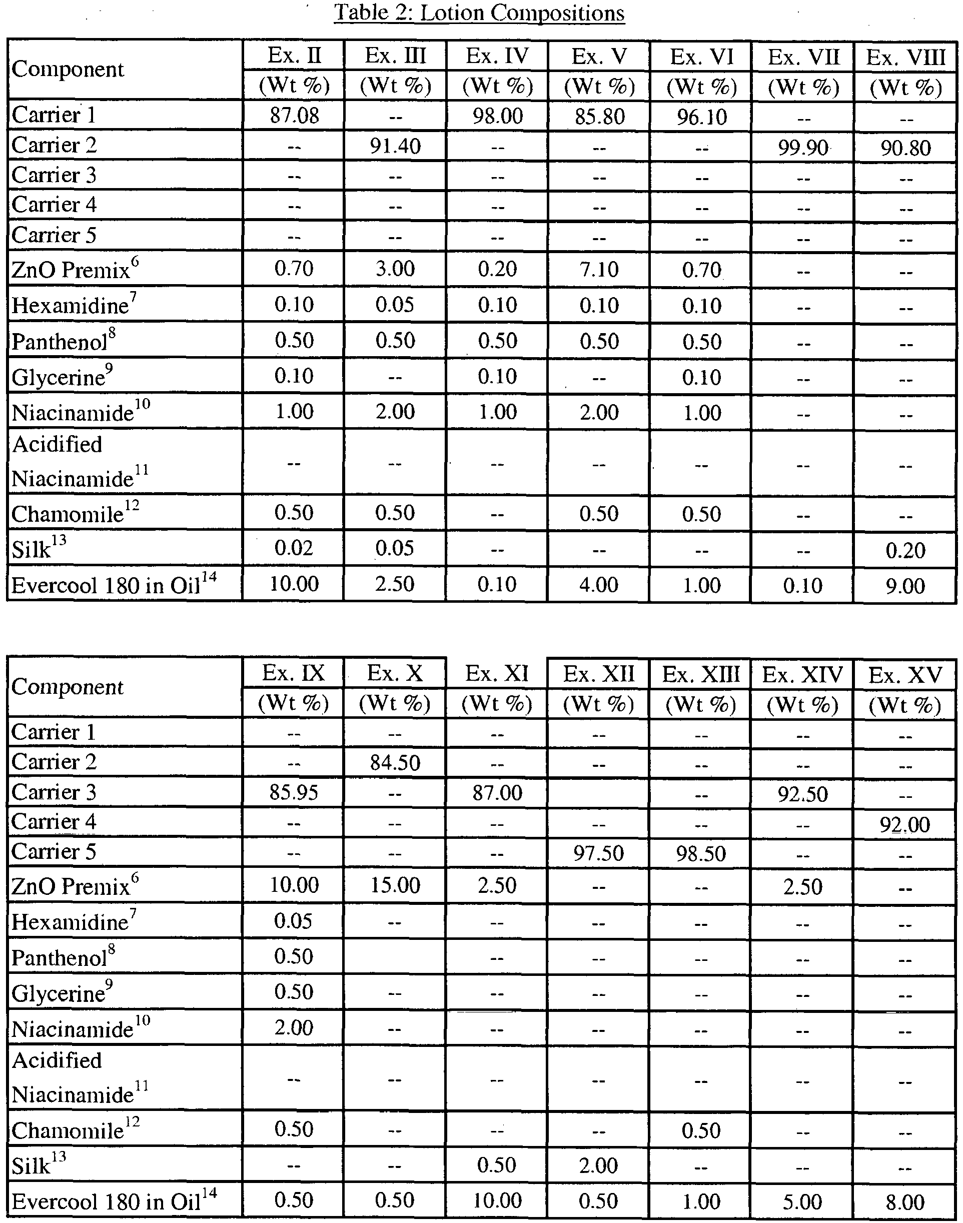

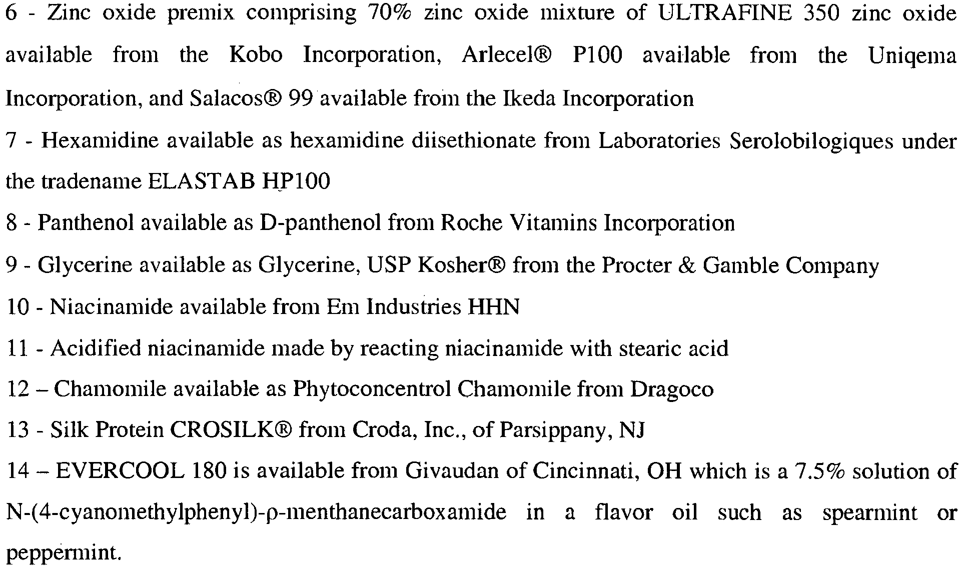

The lotion composition of Example II is subsequently applied to the entire wearer-contacting surface of a DRI-WEAVE topsheet of a sanitary pad product such as Always Wing Regular Long manufactured by the Procter & Gamble Company. To deliver a safe and effective amount of the lotion composition onto the skin, about 0.4 mg/cm² (2.6 mg/in²) of the lotion composition is applied to the topsheet using a Meltex EP45 hot melt applicator having a head operating temperature of about 90°C.

The lotion composition of Example III is subsequently applied by spraying the composition onto the entire wearer-contacting surface of a DRI-WEAVE topsheet of a sanitary pad product such as Envive Miniform manufactured by the Procter & Gamble Company. To deliver a safe and effective amount of the lotion composition onto the skin, about 4.0 mg/cm² (25.8 mg/in²) of the lotion composition is applied to the topsheet using a hot melt pneumatic Dynatec E84B1758 spray head having a head operating temperature of about 90°C and an atomization pressure of about 16 kiloPascals (kPa).

The lotion composition of Example IV is subsequently applied by slot coating (Nordsen EP 11-12-02) striped configurations of the composition onto the wearer-contacting surface of a hydrophobic spunbond bicomponent polyethylene / polypropylene topsheet (BBA, Washougal, WA) of a sanitary pad product. To deliver a safe and effective amount of the lotion composition onto the skin, the lotion composition is applied to the topsheet in a striped configuration wherein the striped configuration comprises at least two stripes each being 40 millimeters (mm) wide x 200 mm long and having about 0.8 mg/cm² (5.2 mg/in²) of the composition applied thereon.

The lotion composition of Example V is subsequently applied by spraying striped configurations of the composition onto the wearer-contacting surface of a DRI-WEAVE topsheet of a panty liner product such as Alldays Regular manufactured by the Procter & Gamble Company. To deliver a safe and effective amount of the lotion composition onto the skin, the lotion composition is applied to the topsheet in a striped configuration wherein the striped configuration comprises at least two stripes each being 40 millimeters (mm) wide x 200 mm long and having about 0.6 mg/cm² (3.9 mg/in²) of the composition applied thereon. The lotion composition is applied to the topsheet using a hot melt pneumatic Dynatec E84B1758 spray head having a head operating temperature of about 90°C and an atomization pressure of about 16 kiloPascals (kPa).

The lotion composition of Example VI is subsequently applied to the entire wearer-contacting surface of a DRI-WEAVE topsheet of a panty liner product such as Alldays Regular manufactured by the Procter & Gamble Company. To deliver a safe and effective amount of the lotion composition onto the skin, about 0.2 mg/cm² (1.3 mg/in²) of the lotion composition is applied to the topsheet using a Meltex EP45 hot melt applicator having a head operating temperature of about 90°C.

The lotion composition of Example VII is subsequently applied by spraying the composition onto the entire wearer-contacting surface of a DRI-WEAVE topsheet of sanitary pad product such as Envive Miniform manufactured by the Procter & Gamble Company. To deliver a safe and effective amount of the lotion composition onto the skin, about 1.0 mg/cm² (6.5 mg/in²) of the lotion composition is applied to the topsheet using a hot melt pneumatic Dynatec E84B1758 spray head having a head operating temperature of about 90°C and an atomization pressure of about 16 kiloPascals (kPa).

The lotion composition of Example VIII is subsequently applied to the entire wearer-contacting surface of a DRI-WEAVE topsheet of a panty liner product such as Alldays Regular manufactured by the Procter & Gamble Company. To deliver a safe and effective amount of the lotion composition onto the skin, about 0.4 mg/cm² (2.6 mg/in²) of the lotion composition is applied to the topsheet using a Meltex EP45 hot melt applicator having a head operating temperature of about 90°C.

The lotion composition of Example IX is subsequently applied by slot coating (Nordsen EP 11-12-02) striped configurations of the composition onto the wearer-contacting surface of a hydrophobic spunbond bicomponent polyethylene / polypropylene topsheet (BBA, Washougal, WA) of a sanitary pad product. To deliver a safe and effective amount of the lotion composition onto the skin, about 3.0 mg/cm² (19.5 mg/in²) of the lotion composition is applied to the topsheet.

The lotion composition of Example X is subsequently applied by spraying the composition onto the entire wearer-contacting surface of a DRI-WEAVE topsheet of sanitary pad product such as Envive Miniform manufactured by the Procter & Gamble Company. To deliver a safe and effective amount of the lotion composition onto the skin, about 1.0 mg/cm² (6.5 mg/in²) of the lotion composition is applied to the topsheet using a hot melt pneumatic Dynatec E84B1758 spray head having a head operating temperature of about 90°C and an atomization pressure of about 16 kiloPascals (kPa).

The lotion composition of Example XI is subsequently applied by spraying the composition onto the hydrophobic spunbond bicomponent polyethylene / polypropylene topsheet (BBA, Washougal, WA) of a sanitary pad product such as Naturella manufactured by the Procter & Gamble Company. To deliver a safe and effective amount of the lotion composition onto the skin, about 1.0 mg/cm² (6.5 mg/in²) of the lotion composition is applied to the topsheet using a hot melt pneumatic Dynatec E84B1758 spray head having a head operating temperature of about 90°C and an atomization pressure of about 16 kiloPascals (kPa).

The lotion composition of Example XII is subsequently applied by spraying the composition onto the hydrophobic spunbond bicomponent polyethylene / polypropylene topsheet (BBA, Washougal, WA) of a sanitary pad product such as Naturella manufactured by the Procter & Gamble Company. To deliver a safe and effective amount of the lotion composition onto the skin, the lotion composition is applied to the topsheet in a striped configuration wherein the striped configuration comprises at least two stripes each being 40 millimeters (mm) wide x 100 mm long and having about 1.2 mg/cm² (7.8 mg/in²) of the composition applied thereon. The lotion composition is applied to the topsheet using a hot melt pneumatic Dynatec E84B1758 spray head having a head operating temperature of about 90°C and an atomization pressure of about 16 kiloPascals (kPa).

The lotion composition of Example XIII is subsequently applied by slot coating (Nordsen EP 11-12-02) striped configurations of the composition onto the wearer-contacting surface of a hydrophobic spunbond bicomponent polyethylene / polypropylene topsheet (BBA, Washougal, WA) of a sanitary pad product. To deliver a safe and effective amount of the lotion composition onto the skin, the lotion composition is applied to the topsheet in a striped configuration wherein the striped configuration comprises at least four stripes each being 5 millimeters (mm) wide x 150 mm long and having about 0.8 mg/cm² (5.2 mg/in²) of the composition applied thereon.

The lotion composition of Example XIV is subsequently applied by slot coating (Nordsen EP 11-12-02) striped configurations of the composition onto the wearer-contacting surface of a hydrophobic spunbond bicomponent polyethylene polypropylene topsheet (BBA, Washougal, WA) of a sanitary pad product. To deliver a safe and effective amount of the lotion composition onto the skin, the lotion composition is applied to the topsheet in a striped configuration wherein the striped configuration comprises at least two stripes each being 15 millimeters (mm) wide x 150 mm long and having about 0,4 mg/cm² (2,6 mg/in²) of the composition applied thereon.

The lotion composition of Example XV is subsequently applied by spraying the composition onto the hydrophobic spunbond bicomponent polyethylene / polypropylene topsheet (BBA, Washougal, WA) of a sanitary pad product. To deliver a safe and effective amount of the lotion composition onto the skin, the lotion composition is applied to the topsheet in a striped configuration wherein the striped configuration comprises at least two stripes each being 20 millimeters (mm) wide x 120 mm long and having about 1.2 mg/cm² (7.8 mg/in²) of the composition applied thereon. The lotion composition is applied to the topsheet using a hot melt pneumatic Dynatec E84B1758 spray head having a head operating temperature of about 90°C and an atomization pressure of about 16 kilopascals (kPa).

The dimensions and values disclosed herein are not to be understood as being strictly limited to the exact numerical values recited. Instead, unless otherwise specked, each such dimension is intended to mean both the recited value and a functionally equivalent range surrounding that value. For example, a dimension disclosed as "40 mm" is intended to mean "about 40 mm."

## Claims

1. An absorbent article comprising:
a topsheet;
a backsheet;
an absorbent core disposed between said topsheet and said backsheet; and
a lotion composition disposed on one or more layers of said absorbent article, said lotion composition comprising:
a carrier; and
a cooling agent comprising a N-substituted p-menthane carboxamide material selected from the group consisting of N-(4-cyanomethylphenyl) p-menthanecarboxamide, N-(4-sulfamoylphenyl) p-menthanecarboxamide, N-(4-cyanophenyl) p-menthanecarboxamide, N-(4-acetylphenyl) p-menthanecarboxamide, N-(4-hydroxymethylphenyl) p-menthanecarboxamide, N-(3-hydroxy-4-methoxyphenyl) p-menthanecarboxamide, and mixtures thereof.

2. The absorbent article of claim 1, wherein said N-substituted p-menthane carboxamide material is N-(4-cyanomethylphenyl)-p-menthanecarboxamide.

3. The absorbent article of any one of the preceding claims, wherein said cooling agent further comprises a cooling material selected from the group consisting of menthol, menthone, isopulegol, N-ethyl p-menthanecarboxamide, N,2,3-trimethyl-2-isopropylbutanamide, menthyl lactate, menthone glycerine acetal, mono-menthyl succinate, mono-menthyl glutarate, O-menthyl glycerine, menthyl-N,N-dimethylsuccinamate, 2-sec-butylcyclohexanone, and mixtures thereof.

4. The absorbent article of any one of the preceding claims, wherein said cooling agent further comprises a cooling material having an enthalpy of vaporization of less than 71 kJ/mol at 101.325 kPa (760 Torr).

5. The absorbent article of any one of the preceding claims, wherein said absorbent article further comprises indicia comprising a color selected from the group consisting of green, blue, and mixtures thereof.

6. The absorbent article of any one of the preceding claims, wherein said lotion composition further comprises an essential oil selected from the group consisting of Acorus gramineus, Anthemis nobilis, Artemisia dracunculus, Basil, Bergamot, Calamintha sylvatica, Caraway, Cedarwood, Chamomile, Cinnamon, Cinnamon bark, Citrus aurantium, Clove, Cypress, Dill, Eucalyptus, Frankincense, Galangol, Geranium, Ginger, Hop, Jasmine, Laurus nobilis, Lavender, Lemon balm, Lemongrass, Lemon, Limonene, Linalool, Linalyl acetate, Lippia alba, Marjoram, Melissa, Myrrh, Neroli, Nutmeg, Passiflora, Patchouli, Peppermint, Pinene, Rose, Rosewood, Rosemary, Sage, Sandalwood, Spearmint, Sweet Fennel, Sweet Orange, Thyme, Valerian, Ylang ylang, and mixtures thereof.

7. The absorbent article of any one of the preceding claims, wherein said carrier is selected from the group consisting of petroleum-based hydrocarbons having from about 4 to about 32 carbon atoms, fatty alcohols having from about 12 to about 24 carbon atoms, polysiloxane compounds, fatty acid esters, alkyl ethoxylates, lower alcohols having from about 1 to about 6 carbon atoms, low molecular weight glycols and polyols, fatty alcohol ethers having from about 12 to about 28 carbon atoms in their fatty chain, lanolin, lanolin derivatives, glycerides, glyceride derivatives including acetoglycerides and ethoxylated glycerides of C₁₂-C₂₈ fatty acids, and mixtures thereof.

8. The absorbent article of any one of the preceding claims, wherein said carrier comprises petrolatum.

9. The absorbent article of any one of the preceding claims, wherein said cooling agent is substantially odorless.

## Patentansprüche

1. Absorptionsartikel, umfassend:
eine Oberschicht,
eine Unterschicht,
einen Absorptionskern, der zwischen der Oberschicht und der Unterschicht angeordnet ist, und
eine Lotionszusammensetzung, die auf einer oder mehreren Schichten des Absorptionsartikels angeordnet ist, wobei die Lotionszusammensetzung Folgendes umfasst:
einen Träger und
ein Kühlmittel, umfassend ein N-substituiertes p-Menthancarboxamid-Material, ausgewählt aus der Gruppe bestehend aus N-(4-Cyanomethylphenyl)-p-menthancarboxamid, N-(4-Sulfamoylphenyl)-p-menthancarboxamid, N-(4-Cyanophenyl)-p-menthancarboxamid, N-(4-Acetylphenyl)-p-menthancarboxamid, N-(4-Hydroxymethylphenyl)-p-menthancarboxamid, N-(3-Hydroxy-4-methoxyphenyl)-p-menthancarboxamid und Mischungen davon.

2. Absorptionsartikel nach Anspruch 1, wobei das N-substiW ierte p-Menthancarboxamid-Material N-(4-Cyanometlrylphenyl)-p-menthancarboxamid ist.

3. Absorptionsartikel nach einem der vorstehenden Ansprüche, wobei das Kühlmittel ferner ein Kühlmaterial umfasst, das ausgewählt ist aus der Gruppe bestehend aus Menthol, Menthon, Isopulegol, N-Etlryl-p-menthancarboxamid, N,2,3-Trimethyl-2-isopropylbutanamid, Menthyllactat, Menthonglycerinacetal, Monomenthylsuccinat, Monomentlrylglutarat, O-Mentlrylglycerin, Menthyl-N,N-dimethylsuccinamat, 2-sek.-Butylcyclohexanon und Mischungen davon.

4. Absorptionsartikel nach einem der vorstehenden Ansprüche, wobei das Kühlmittel ferner ein Kühlmaterial umfasst, das eine Verdampfungsenthalpie von weniger als 71 kJ/mol bei 101,325 kPa (760 Torr) aufweist.

5. Absorptionsartikel nach einem der vorstehenden Ansprüche, wobei der Absorptionsartikel ferner Markierungen umfasst, die eine Farbe umfassen, ausgewählt aus der Gruppe bestehend aus Grün, Blau und Mischungen davon.

6. Absorptionsartikel nach einem der vorstehenden Ansprüche, wobei die Lotionsziisanumensetzimg ferner ein ätherisches Öl umfasst, ausgewählt aus der Gruppe bestehend aus Acorus gramineus, Anthemis nobilis, Artemisia dracunculus, Basilikum, Bergamotte, Calamintha sylvatica, Kümmel, Zedernholz, Kamille, Zimt, Zimtrinde, Citrus aurantium, Gewürznelke, Zypresse, Dill, Eukalyptus, Weihrauch, Galangol, Geranie, Ingwer, Hopfen, Jasmin, Laurus nobilis, Lavendel, Zitronenmelisse, Zitronengras, Zitrone, Limonen, Linalool, Linalylacetat, Lippia alba, Majoran, Melisse, Myrrhe, Neroli, Muskat, Passiflora, Patschuli, Pfefferminze, Pinen, Rose, Palisander, Rosmarin, Salbei, Sandelholz, Grüner Minze, Süßem Fenchel, Süßer Orange, Thymian, Baldrian, Ylang-Ylang und Mischungen davon.

7. Absorptionsartikel nach einem der vorstehenden Ansprüche, wobei der Träger ausgewählt ist aus der Gruppe bestehend aus Petroleum-basierten Kohlenwasserstoffen mit etwa 4 bis etwa 32 Kohlenstoffatomen, Fettalkoholen mit etwa 12 bis etwa 24 Kohlenstoffatomen, Polysiloxanverbindungen, Fettsäureestern, Alkylethoxylaten, niederen Alkoholen mit etwa 1 bis etwa 6 Kohlenstoffatomen, niedermolekularen Glycolen und Polyolen, Fettalkoholethern mit etwa 12 bis etwa 28 Kohlenstoffatomen in ihrer Fettkette, Lanolin, Lanolinderivaten, Glyceriden, Glyceridderivaten, einschließlich Acetoglyceriden und ethoxylierten Glyceriden von C₁₂-C₂₈-Fettsäuren, und Mischungen davon.

8. Absorptionsartikel nach einem der vorstehenden Ansprüche, wobei der Träger Petrolatum umfasst.

9. Absorptionsartikel nach einem der vorstehenden Ansprüche, wobei das Kühlmittel im Wesentlichen geruchlos ist.

## Revendications

1. Article absorbant comprenant :
une feuille de dessus ;
une feuille de fond ;
une âme absorbante disposée entre ladite feuille de dessus et ladite feuille de fond ; et
une composition de lotion disposée sur une ou plusieurs couches dudit article absorbant, ladite composition de lotion comprenant :
un véhicule, et
un agent rafraîchissant comprenant un matériau p-menthane carboxamide N-substitué choisi dans le groupe constitué de N-(4-cyanométhylphényl)-p-menthanecarboxamide, N-(4-sulfamoylphényl)-p-menthanecarboxamide, N-(4-cyanophényl)-p-menthanecarboxamide, N-(4-acétylphényl)-p-menthanecarboxamide, N-(4-hydroxyméthylphényl)-p-menthanecarboxamide, N-(3-hydroxy-4-méthoxyphényl)-p-menthanecarboxamide, et leurs mélanges.

2. Article absorbant selon la revendication 1, dans lequel le matériau p-menthane carboxamide N-substitué est le N-(4-cyanométhylphényl)-p-menthanecarboxamide.

3. Article absorbant selon l'une quelconque des revendications précédentes, dans lequel ledit agent rafraîchissant comprend en outre un matériau rafraîchissant choisi dans le groupe constitué de menthol, menthone, isopulégol, N-éthyl-p-menthanecarboxamide, N,2,3-triméthyl-2-isopropylbutanamide, lactate de menthyle, menthone glycérine acétal, mono-menthylsuccinate, mono-menthylglutarate, O-menthyle glycérine, menthyl-N,N-diméthylsuccinamate, 2-sec-butylcyclohexanone, et leurs mélanges.

4. Article absorbant selon l'une quelconque des revendications précédentes, dans lequel ledit agent rafraîchissant comprend en outre un matériau rafraîchissant ayant une enthalpie de vaporisation inférieure à 71 kJ/mol sous une pression de 101,325 kPa (760 Torr).

5. Article absorbant selon l'une quelconque des revendications précédentes, dans lequel ledit article absorbant comprend en outre des marquages comprenant une couleur choisie dans le groupe constitué du vert, du bleu et de leurs mélanges.

6. Article absorbant selon l'une quelconque des revendications précédentes, dans lequel ladite composition de lotion comprend en outre une huile essentielle choisie dans le groupe constitué de Acorns gramineus, Anthemis nobilis, Artemisia dracunculus, basilic, bergamote, Calamintha sylvatica, Carum carvi, bois de cèdre, camomille, cannelle, écorce de cannelle, Citrus aurantium, clou de girofle, cyprès, aneth, eucalyptus, encens, galangol, géranium, gingembre, houblon, jasmin, Laurus nobilis, lavande, baume de citron, citronelle, citron, limonène, linalol, acétate de linalyle, Lippia alba, marjolaine, mélisse, myrrhe, Neroli, noix de muscade, passiflore, patchouli, menthe poivrée, pinène, rose, bois de rose, romarin, sauge, bois de santal, menthe verte, fenouil doux, orange douce, thym, valériane, Ylang ylang, et leurs mélanges.

7. Article absorbant selon l'une quelconque des revendications précédentes, dans lequel ledit véhicule est choisi dans le groupe constitué d'hydrocarbures à base de pétrole ayant d'environ 4 à environ 32 atomes de carbone, alcools gras ayant d'environ 12 à environ 24 atomes de carbone, composés polysiloxanes, esters d'acides gras, éthoxylates d'alkyle, alcools inférieurs ayant d'environ 1 à environ 6 atomes de carbone, glycols et polyols à bas poids moléculaire, éthers d'alcools gras ayant d'environ 12 à environ 28 atomes de carbone dans leur chaîne grasse, lanoline, dérivés de lanoline, glycérides, dérivés glycéridiques y compris les acétoglycérides et les glycérides éthoxylés d'acides gras en C₁₂ à C₂₈, et leurs mélanges.

8. Article absorbant selon l'une quelconque des revendications précédentes, dans lequel le véhicule comprend de la vaseline.

9. Article absorbant selon l'une quelconque des revendications précédentes, dans lequel ledit agent rafraîchissant est sensiblement inodore.
